# EUROPEAN PATENT APPLICATION

(11) **EP 4 307 312 A1**
(43) Date of publication of application: **17.01.2024**
(21) Application number: 22767204.5
(22) Date of filing: 09.03.2022
(51) Int. Cl.: G16H 20/10, G16H 70/40, G16Y 10/60

(54) **PATIENT FOLLOW-UP ASSISTANCE SYSTEM, PATIENT FOLLOW-UP ASSISTANCE METHOD, ASSISTANCE CONTROL DEVICE, TERMINAL DEVICE, AND PROGRAM STORAGE MEDIUM**

(30) Priority: 09.03.2021 JP 2021037596
(71) Applicant: NTT Communications Corporation, Tokyo 100-8019 (JP)
(72) Inventor: TSUNEKAWA, Satoshi, Tokyo 100-8019 (JP); KONAGAYA, Takahito, Tokyo 100-8019 (JP); SAKURAI, Yoichi, Tokyo 100-8019 (JP); IZUMI, Hiroaki, Tokyo 100-8019 (JP); TABATA, Masaaki, Tokyo 100-8019 (JP)
(74) Representative: Gramm, Lins & Partner Patent- und Rechtsanwälte PartGmbB
(86) International application number: PCT/JP2022/010379
(87) International publication number: WO 2022/191250

(57) **Abstract**

According to an aspect of the present invention, a terminal device transmits administration completion information to a support control apparatus together with unique identification information of the patient. The support control apparatus receives the administration completion information and stores it in association with the unique identification information, extracts a patient corresponding to an extraction condition as a research target person based on the administration completion information and a preset extraction condition, and transmits a request for response to the terminal device of the extracted research target person. The terminal device returns response information representing a condition after medicine administration input by the patient in response to the request for response to the support control apparatus together with the unique identification information. The support control apparatus estimates a condition change of the patient after the medicine administration based on the response information, and outputs the result.

## Description

### FIELD

Embodiments described herein relate generally to a patient follow-up research support system and a patient follow-up research support method having a function of supporting follow-up research on a condition of a patient who has received, for example, administration of a medicine, and a support control apparatus, a terminal device, and a program storage medium for use in the patient follow-up research support system.

### BACKGROUND

In the medical or pharmaceutical field, so-called clinical trials are carried out in which the conditions of patients after medication are followed and researched. In conventional clinical trials, for example, inpatients or outpatients are subjects, and generally, a clinical trial coordinator extracts clinical trial subjects on the basis of medical records or the like of the patients to conduct the clinical trials. For this reason, there is a problem in that much labor and time are required for the work for extracting the clinical trial subjects, and failure to extract the patients who should have been subjects is likely to occur.

Therefore, there has been proposed a system in which prescription and dispensing information of inpatients or outpatients is registered in a database, and the registered prescription and dispensing information is data-collated with clinical trial information indicating a preset clinical trial standard by an information processing apparatus, thereby screening clinical trial subjects and constructing a database of suitable patients for a clinical trial (for example, refer to Patent Literature 1).

### CITATION LIST

### PATENT LITERATURE

(Patent Literature 1) Japanese Patent No. 6338957

### SUMMARY

### TECHNICAL PROBLEM

However, in the system as proposed above, the subjects of clinical trials are limited to patients whose medical care information such as medical records or prescriptions is managed by medical institutions, pharmacies, or the like, such as inpatients and outpatients. Since the number of clinical trials is small, there is a problem in that subjects of clinical trials cannot be extracted from a wider range of patient groups.

In particular, for example, in a case where follow-up research after vaccination is to be performed on vaccinated persons without omission, many healthy persons are included among the vaccinated persons, and medical care information such as medical records or prescriptions is not managed in medical institutions, pharmacies, or the like for those vaccinated persons in many cases. Therefore, it is difficult to perform follow-up research after vaccination with the proposed system described above.

The present invention has been made in view of the above circumstances, and in one aspect, an object of the present invention is to provide a technique that enables follow-up research after medication for a wider range of patients who have received the medication.

### SOLUTION TO PROBLEM

In order to solve the problem described above, an aspect of the present invention relates to a patient follow-up research support system and a patient follow-up research support method for supporting follow-up research on a patient who has received administration of a medicine, the patient follow-up research support system including a terminal device for information registration and a support control apparatus capable of communicating information data with the terminal device via a network.

The terminal device first accepts an input of administration completion information issued to the patient who has received administration of the medicine, and transmits the administration completion information to the support control apparatus together with unique identification information of the patient. On the other hand, the support control apparatus receives the administration completion information transmitted from the terminal device and stores the received administration completion information in association with the unique identification information. Then, the support control apparatus extracts, based on the administration completion information and a preset extraction condition, a patient corresponding to the extraction condition as a research target person and transmits a request for response to the terminal device corresponding to the extracted research target person. Upon receipt of the request for response, the terminal device returns response information representing a condition after the medicine administration input by the patient in response to the request for response to the support control apparatus together with the unique identification information of the patient. After receiving and storing the transmitted response information, the support control apparatus estimates a condition change of the patient after the medicine administration based on the response information, and outputs the estimation result.

### ADVANTAGEOUS EFFECTS OF INVENTION

According to one aspect of the present invention, it is possible to provide a technique that enables follow-up research after medication for a wider range of patients who have received medication.

### BRIEF DESCRIPTION OF THE DRAWINGS

FIG. 1 is a diagram showing an example of an overall configuration including a vaccinated person follow-up support apparatus which is an embodiment of a patient follow-up support apparatus according to the present invention, peripheral systems thereof, and vaccinated person terminals.
FIG. 2 is a block diagram showing a hardware configuration of a vaccinated person terminal according to the embodiment of the present invention.
FIG. 3 is a block diagram showing an example of a software configuration of the vaccinated person terminal shown in FIG. 2.
FIG. 4 is a block diagram showing a hardware configuration of a vaccinated person information management server included in the vaccinated person follow-up support apparatus according to the embodiment of the present invention.
FIG. 5 is a block diagram showing an example of a software configuration of the vaccinated person information management server shown in FIG. 4.
FIG. 6 is a block diagram showing an example of a software configuration of a medical care information management server included in the vaccinated person follow-up support apparatus according to the embodiment of the present invention.
FIG. 7 is a block diagram showing an example of a software configuration of a response server included in the vaccinated person follow-up support apparatus according to the embodiment of the present invention.
FIG. 8 is a flowchart showing an example of procedures and details of processing executed by the vaccinated person terminal shown in FIG. 3.
FIG. 9 is a flowchart showing an example of procedures and details of processing executed by the vaccinated person information management server shown in FIG. 5.
FIG. 10 is a flowchart showing an example of procedures and details of processing executed by the medical care information management server shown in FIG. 6.
FIG. 11 is a flowchart showing an example of procedures and details of processing executed by the response server shown in FIG. 7.
FIG. 12 is a sequence diagram showing a flow of data between the vaccinated person follow-up support apparatus and each of its peripheral systems and vaccinated person terminals shown in FIG. 1.

### DETAILED DESCRIPTION

Embodiments of the present invention will be described below with reference to the drawings.

### SUMMARY OF THE INVENTION

According to an aspect of the present invention, a terminal device accepts an input of administration completion information issued to a patient who has received administration of a medicine, and transmits the administration completion information to a support control apparatus together with unique identification information of the patient. On the other hand, the support control apparatus receives the administration completion information and stores it in association with the unique identification information, extracts a patient corresponding to an extraction condition as a research target person based on the administration completion information and a preset extraction condition, and transmits a request for response to the terminal device of the extracted research target person. The terminal device returns response information representing a condition after medicine administration input by the patient in response to the request for response to the support control apparatus together with the unique identification information. After receiving and storing the response information, the support control apparatus estimates a condition change of the patient after the medicine administration based on the response information, and outputs the result.

According to the aspect of the invention, for example, even for a patient whose medical record or the like is not managed in a medical institution or the like, it is possible to register the administration completion information of the patient in the support control apparatus by using the terminal device of the patient. Therefore, the support control apparatus can manage a wider range of patients.

In addition, since the research target patients are extracted based on the acquired administration completion information and the response information is acquired from the terminal devices of these patients, it is possible to efficiently acquire the response information from many patients in a short period of time. Then, the support control apparatus performs analysis processing on the acquired response information in real time, so that it is possible to efficiently analyze the post-vaccination situations of more vaccinated persons within an appropriate period and output the results.

As a result, for example, a medical institution, a pharmaceutical company, or a local government can perform statistical processing related to side reactions and the like after medicine administration, on-line medical care, face-to-face medical care, or the like at an appropriate timing.

### <Embodiment>

### (Configuration Example)

### (1) System

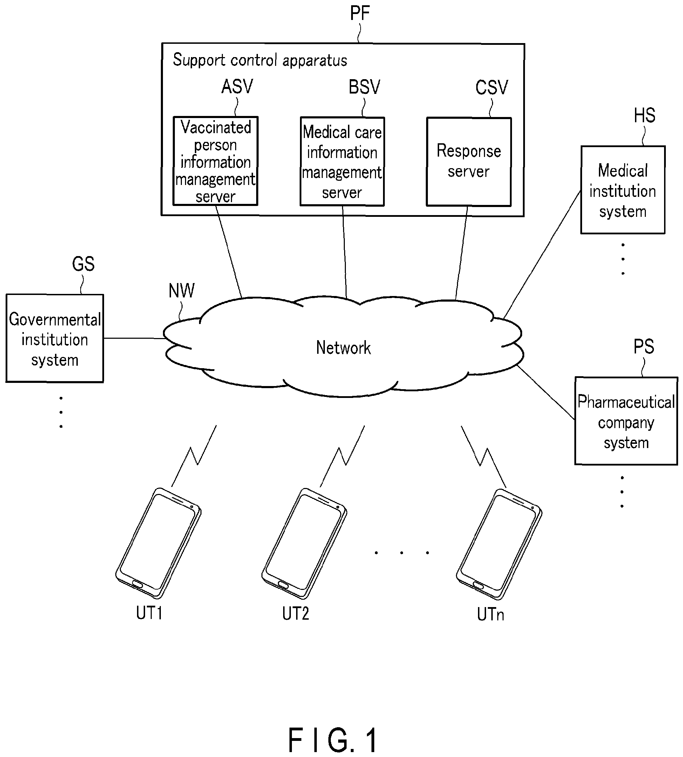

FIG. 1 is a diagram showing an example of an overall configuration of a vaccinated person follow-up research support system which is an embodiment of a patient follow-up research support system according to the present invention, PF indicating a support control apparatus which is a core of the system. The support control apparatus PF is hereinafter also referred to as a platform.

The support control apparatus PF includes a vaccinated person information management server ASV, a medical care information management server BSV, and a response server CSV. These servers ASV, BSV, and CSV constitute a platform for realizing functions of the vaccinated person follow-up research support apparatus.

The vaccinated person information management server ASV, the medical care information management server BSV, and the response server CSV can perform data transmission with a system HS operated by a medical institution, a system PS operated by a pharmaceutical company, and a system GS operated by a governmental institution such as a local government via a network NW. The vaccinated person information management server ASV, the medical care information management server BSV, and the response server CSV can also perform data transmission with vaccinated person terminals UT1 to UTn (hereinafter collectively referred to as UT) used by the vaccinated persons via the network NW.

Each of the system HS operated by the medical institution, the system PS operated by the pharmaceutical company, and the system GS operated by the governmental institution includes a server computer including a database and an administrator terminal used by a person in charge of research. A medical care worker and a person in charge of research of the pharmaceutical company and the local government acquire and manage various kinds of information using their respective administrator terminals.

The network NW includes, for example, a wide area network having the Internet as a core and an access network for accessing the wide area network. As the access network, a public wired or wireless communication network, a local wired or wireless area network (LAN), and a cable television (CATV) network are used.

### (2) Apparatus

### (2-1) Vaccinated Person Terminal UT

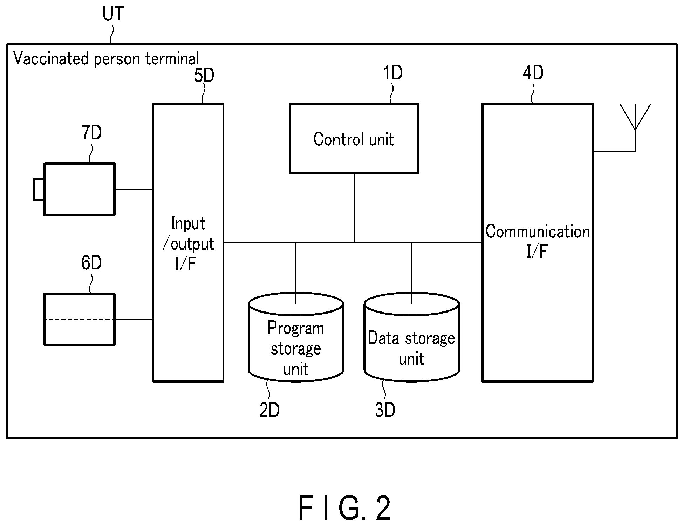

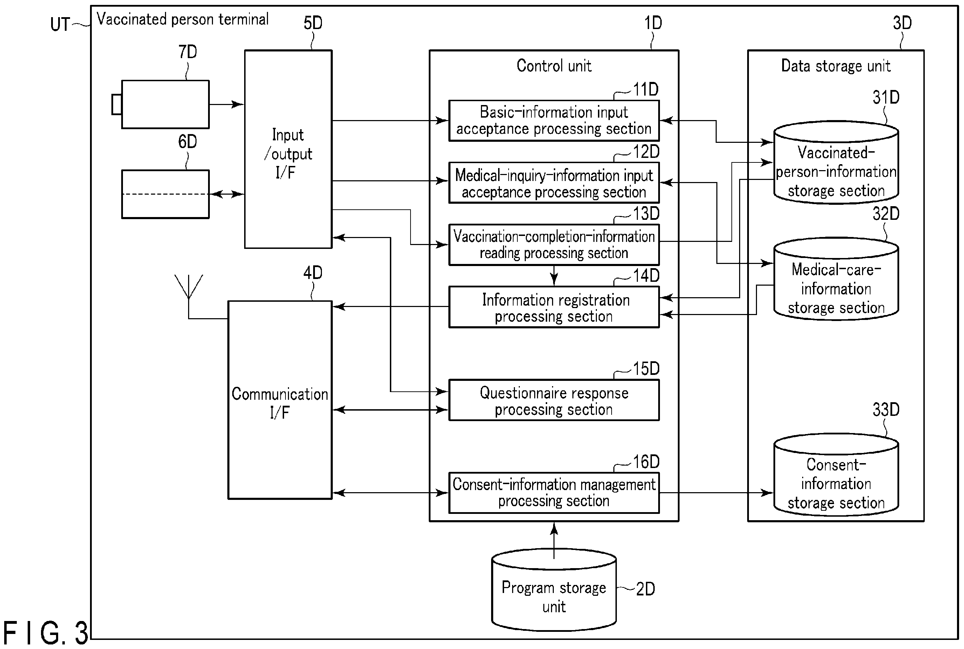

FIGS. 2 and 3 are block diagrams respectively showing examples of a hardware configuration and a software configuration of a vaccinated person terminal UT.

The vaccinated person terminal UT is composed of, for example, a general-purpose smartphone including a camera, a browser, and an application for transferring information data using email, a social network system (SNS), a short message service (SMS), or the like. As the vaccinated person terminal UT, a tablet terminal, a wearable terminal, a notebook personal computer, or the like may be used as long as it has a similar function.

The vaccinated person terminal UT includes a control unit 1D using a hardware processor such as a central processing unit (CPU). The control unit 1D is connected to a storage unit including a program storage unit 2D and a data storage unit 3D, a communication interface ("interface" is hereinafter referred to as "I/F") 4D, and an input/output I/F 5D.

Under the control of the control unit 1D, using a communication protocol defined by the network NW, the communication I/F 4D communicates with the vaccinated person information management server ASV, the medical care information management server BSV, and the response server CSV.

The input/output I/F 5D is connected to an input/output device 6D and a camera 7D. The input/output device 6D is composed of, for example, a display device using liquid crystal or organic EL, and an input device using a touch-type input sheet of a pressure-sensitive type or a capacitance type disposed on a display screen of the display device. The input device is used by the vaccinated person to input various kinds of information such as basic information SS1 and consent confirmation information of the vaccinated person, medical inquiry information SS2, and response information on a questionnaire. The display device is used for displaying display data such as template data and questionnaire data to support input of each of the above data.

The camera 7D is used, for example, for optically reading vaccination completion information SS4 marked on a vaccination completion seal or sheet received at the vaccination site, for example, when the person was vaccinated. The vaccination completion information SS4 is represented by code data such as a bar code or a QR code (registered trademark), but may be represented by a plain text so that the vaccinated person can directly confirm the vaccination completion information SS4.

In addition, the input/output I/F 5D may be connected to a global positioning system (GPS) sensor for measuring the position of the vaccinated person or a vital sensor for measuring biological information such as the body temperature, blood pressure, and heart rate of the vaccinated person.

As a storage medium, the program storage unit 2D is configured, for example, to be a combination of a nonvolatile memory, such as a solid state drive (SSD), for which writing and reading can be performed at any time, and a nonvolatile memory, such as a read only memory (ROM). In addition to middleware such as an operating system (OS), the program storage unit 2D stores application programs necessary to execute various controlling processes relating to the embodiment. Hereinafter, the OS and the application programs are collectively referred to as programs.

As a storage medium, the data storage unit 3D is, for example, a combination of a nonvolatile memory, such as an SSD, for which writing and reading can be performed at any time, and a volatile memory, such as a RAM (Random Access Memory). The data storage unit 3D includes, as main storage sections necessary to implement the embodiment, a vaccinated-person-information storage section 31D, a medical-care-information storage section 32D, and a consent-information storage section 33D.

The vaccinated-person-information storage section 31D is used for storing the basic information SS1 and the vaccination completion information SS4 of the vaccinated person in association with unique identification information of the vaccinated person. The basic information SS1 includes, for example, attribute information such as the name, age, and address of the vaccinated person, and contact information such as a phone number or mail address. The vaccination completion information SS4 includes, for example, the type of the inoculated vaccine, the identification information (manufacturer ID) of the pharmaceutical company, and the lot number, but may also include a vaccinated person identification number, a vaccination date and time, a vaccination place, and the like. The vaccinated person information is also referred to as a vaccinated person notebook.

The medical-care-information storage section 32D is used for storing medical care information of the vaccinated person. The medical care information includes, for example, medical inquiry information SS2, and may also include personal health record (PHR) information of the vaccinated person, and prescription/medication information included in an electronic medication notebook or the like.

The consent-information storage section 33D is used for storing information indicating whether or not the vaccinated person has consented to the use of the vaccinated person information and the medical care information by the research organization such as a medical institution, a pharmaceutical company, and a local government.

The control unit 1D includes, as processing functions necessary for implementing the embodiment, a basic-information input acceptance processing section 11D, a medical-inquiry-information input acceptance processing section 12D, a vaccination-completion-information reading processing section 13D, an information registration processing section 14D, a questionnaire response processing section 15D, and a consent-information management processing section 16D. Each of these processing sections 11D to 16D is realized by causing the hardware processor of the control unit 1D to execute a program stored in the program storage unit 2D.

A part or all of the processing sections 11D to 16D may be realized by using hardware such as a large scale integration (LSI) or an application specific integrated circuit (ASIC).

For example, when the vaccinated person inputs the basic information SS1 through the input/output device 6D, the basic-information input acceptance processing section 11D causes the display device to display template data, receives the basic information SS1 input via the input device in accordance with the template data, and stores the received information in the vaccinated-person-information storage section 31D.

For example, when a person to be vaccinated inputs the medical inquiry information SS2 through the input/output device 6D prior to vaccination, the medical-inquiry-information input acceptance processing section 12D causes the display device to display template data, receives the medical inquiry information SS2 input via the input device in accordance with the template data, and stores the received information in the medical-care-information storage section 32D. In a case where a medical inquiry sheet made of a paper medium is used, the medical-inquiry-information input acceptance processing section 12D optically reads the medical inquiry sheet with the camera 7D, and stores the read information in the medical-care-information storage section 32D as image data or text data obtained by converting image data with character recognition.

The vaccination-completion-information reading processing section 13D optically reads through the camera 7D the vaccination completion information SS4 indicated on the vaccination completion seal, sheet, or the like received at the vaccination site at the time of vaccination, receives the read vaccination completion information SS4, and stores the received information in the vaccinated-person-information storage section 31D. In a case where the vaccination completion information SS4 is recorded in a storage medium such as a magnetic card or a memory, the vaccination-completion-information reading processing section 13D reads the vaccination completion information SS4 from the storage medium.

Before or immediately after vaccination, the information registration processing section 14D reads out the vaccinated person information stored in the vaccinated-person-information storage section 31D, that is, the basic information SS1 and the vaccination completion information SS4 of the vaccinated person. Then, the read out vaccinated person information is transmitted from the communication I/F 4D to the vaccinated person information management server ASV.

In addition, the information registration processing section 14D reads out the medical care information stored in the medical-care-information storage section 32D, that is, the medical inquiry information SS2, the PHR information and the prescription/medication information of the vaccinated person, and the like in conjunction with the transmission of the vaccinated person information described above. Then, the read medical care information is transmitted from the communication I/F 4D to the medical care information management server BSV.

During the follow-up period after the vaccination, the questionnaire response processing section 15D receives via the communication I/F 4D a request for response to the questionnaire including an access destination uniform resource locator (URL) transmitted from the response server CSV. Then, the questionnaire response processing unit 15D receives via the communication I/F 4D the questionnaire data and consent inquiry information transmitted from the response server CSV in response to an access operation of the vaccinated person to the access destination URL and causes the display device to display the received data.

In addition, the questionnaire response processing section 15D performs processing of returning, to the response server CSV through the communication I/F 4D, response data SS6 and consent confirmation information SS3 input by the vaccinated person in accordance with the questionnaire data via the input device.

The consent-information management processing section 16D performs processing of inquiring whether there is a consent to the vaccinated person in the transmission processing of the vaccinated person information and the medical care information by the information registration processing section 14D and the questionnaire response processing by the questionnaire response processing section 15D, and managing consent confirmation information SS3 indicating the result of the inquiry in the consent-information storage section 33D.

### (2-2) Vaccinated Person Information Management Server ASV

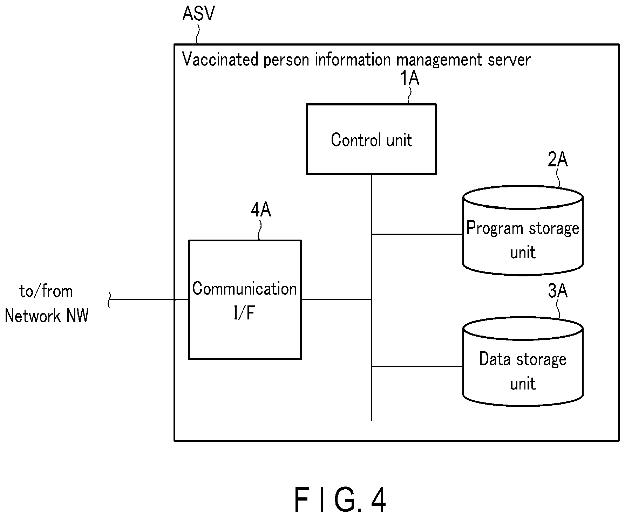

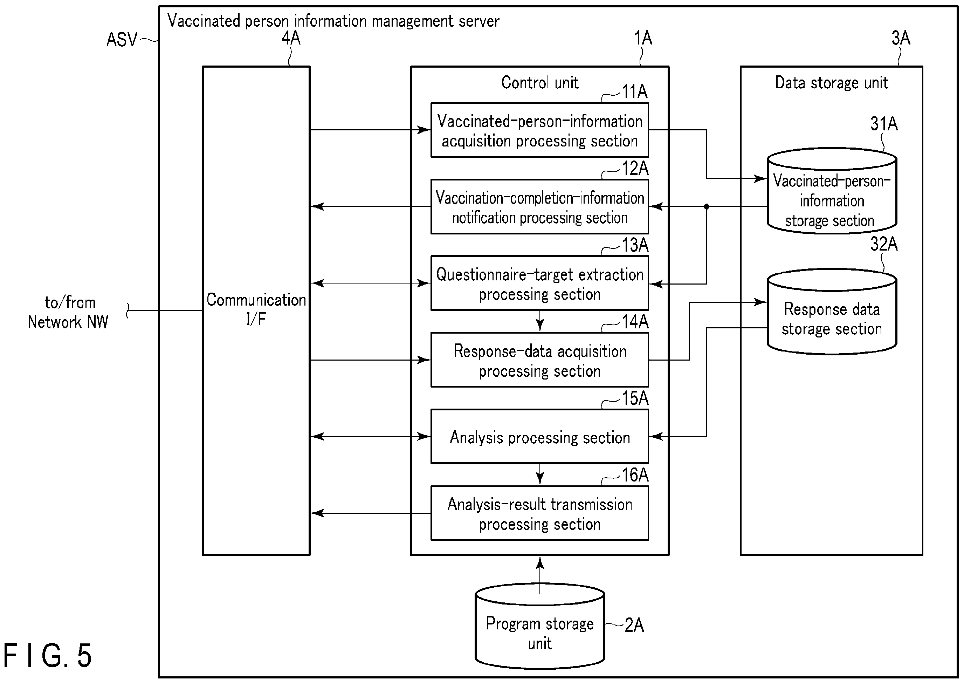

FIGS. 4 and 5 are block diagrams respectively showing examples of a hardware configuration and a software configuration of the vaccinated person information management server ASV.

The vaccinated person information management server ASV is composed of a server computer disposed on a cloud, for example, and includes a control unit 1A using a hardware processor such as a CPU. The control unit 1A is connected to a storage unit including a program storage unit 2A and a data storage unit 3A, and a communication I/F 4A.

Under the control of the control unit 1A, using a communication protocol defined by the network NW, the communication I/F 4A transmits and receives information data to and from the medical care information management server BSV and the response server CSV in the support control apparatus PF, and further to and from the vaccinated person terminal UT, the medical institution system HS, the pharmaceutical company system PS, and the governmental institution system GS.

As a storage medium, the program storage unit 2A is configured, for example, to be a combination of a nonvolatile memory, such as a hard disk drive (HDD), an SSD, or the like, for which writing and reading can be performed at any time, and a nonvolatile memory, such as a ROM. In addition to middleware such as an OS, the program storage section 2A stores programs necessary to execute various controlling processes relating to the embodiment of the present invention.

As a storage medium, the data storage unit 3A is, for example, a combination of a nonvolatile memory, such as an HDD, an SSD, or the like, for which writing and reading can be performed at any time, and a volatile memory, such as a R_AM. The data storage unit 3A includes, as main storage sections necessary to implement the embodiment of the present invention, a vaccinated-person-information storage section 31A and a response data storage section 32A.

The vaccinated-person-information storage section 31A stores the vaccinated person information sent from the vaccinated person terminal UT in a state in which secret sharing processing has been performed. The response data storage section 32A stores the response data SS6 transferred from the response server CSV in a state in which secret sharing processing has been performed.

The control unit 1A includes, as processing functions according to the embodiment of the present invention, a vaccinated-person-information acquisition processing section 11A, a vaccination-completion-information notification processing section 12A, a questionnaire-target extraction processing section 13A, a response-data acquisition processing section 14A, an analysis processing section 15A, and an analysis-result transmission processing section 16A. Each of these processing sections 11A to 16A is realized by causing the hardware processor of the control unit 1A to execute a program stored in the program storage unit 2A.

A part or all of the processing sections 11A to 16A may be realized by using hardware such as an LSI or an ASIC.

The vaccinated-person-information acquisition processing section 11A receives the vaccinated person information transmitted from each of the vaccinated person terminals UT1 to UTn via the communication I/F 4A, performs secret sharing on each of the received vaccinated person information, and then causes the processed information to be stored in the vaccinated-person-information storage section 31A.

The vaccination-completion-information notification processing section 12A reads the vaccination completion information SS4 from the vaccinated-person-information storage section 31A, and transmits the read vaccination completion information SS4 from the communication I/F 4A to the administrator terminal of the corresponding pharmaceutical company system PS or the governmental institution system GS.

For example, every time a predetermined time elapses from the vaccination date, the questionnaire-target extraction processing section 13A performs processing of extracting vaccinated persons who were vaccinated on the vaccination date from the vaccinated-person-information storage section 31A and passing a vaccinated person list of the extracted vaccinated persons to the response-data acquisition processing section 14A. The processing of extracting the vaccinated persons may be performed for each vaccination site.

The response-data acquisition processing section 14A transmits a questionnaire request SS8 including the vaccinated person list of the vaccinated persons extracted by the questionnaire-target extraction processing section 13A to the response server CSV from the communication I/F 4A. In addition, the response-data acquisition processing section 14A receives the response data SS6 sent back from the response server CSV via the communication I/F 4A in response to the questionnaire request SS8, performs secret sharing on the received response data SS6, and causes the processed data to be stored in the response data storage section 32A. That is, as means for acquiring the response data SS6, a technology of electronic Patient Reported Outcome (ePRO) is applied.

The analysis processing section 15A reads the response data SS6 that has been subjected to secret sharing from the response data storage section 32A for each vaccinated person, and acquires the medical care information of the corresponding vaccinated person from the medical care information management server BSV via the network NW. Then, secret calculation processing is performed on the response data SS6 and the medical care information to analyze the contents, thereby estimating, for example, the presence or absence of side reactions and the degree thereof, and the necessity of on-line medical care or face-to-face medical care, and outputting analysis data SS7 including the estimation results to the analysis-result transmission processing section 16A.

The analysis-result transmission processing section 16A transmits the analysis data SS7 output from the analysis processing section 15A from the communication I/F 4A to the medical institution system HS or the pharmaceutical company system PS. The analysis-result transmission processing section 16A may also transmit the analysis data SS7 to the governmental institution system GS or to the vaccinated person terminal UT of the corresponding vaccinated person.

### (2-3) Medical Care Information Management Server BSV

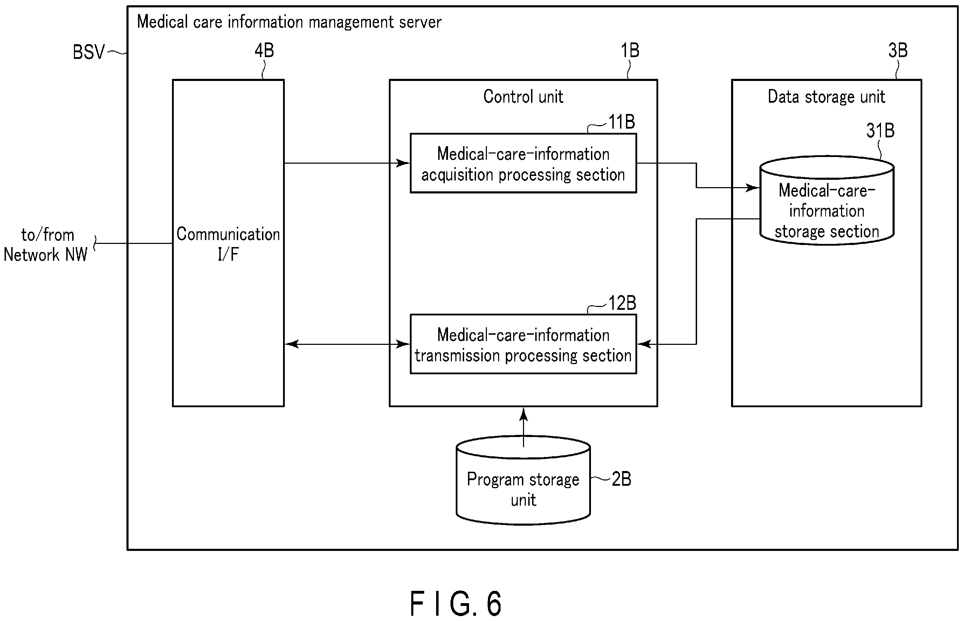

FIG. 6 is a block diagram showing an example of a software configuration of the medical care information management server BSV. Since the hardware configuration is the same as the configuration of the vaccinated person information management server ASV (FIG. 4), the description thereof is omitted here.

The medical care information management server BSV includes a control unit 1B, a program storage unit 2B, a data storage unit 3B, and a communication I/F 4B. The communication I/F 4B performs data transmission and reception between a vaccinated person terminal UT and the vaccinated person information management server ASV via the network NW. The medical care information management server BSV may have a function of transferring the medical care information of the vaccinated person to the medical institution system HS, the pharmaceutical company system PS, or the governmental institution system GS within a range of a predetermined condition (for example, whether there is a consent).

The data storage unit 3B includes a medical-care-information storage section 31B. The medical-care-information storage section 31B stores the medical care information of the vaccinated person transmitted from the vaccinated person terminal UT in a state in which the medical care information has been subjected to secret sharing.

The control unit 1B includes, as processing functions according to the embodiment of the present invention, a medical-care-information acquisition processing section 11B and a medical-care-information transmission processing section 12B. Each of these processing sections 11B and 12B is realized by causing the hardware processor of the control unit 1B to execute a program stored in the program storage unit 2B.

One or both of the processing sections 11B and 12B may be realized by using hardware such as an LSI or an ASIC.

The medical-care-information acquisition processing section 11B receives the medical care information transmitted from each of the vaccinated person terminals UT1 to UTn via the communication I/F 4B, performs secret sharing on each of the received medical care information, and then causes the processed information to be stored in the medical-care-information storage section 31B.

The medical-care-information transmission processing section 12B reads the corresponding medical care information from the medical-care-information storage section 31B in response to a request for acquiring medical care information transmitted from the vaccinated person information management server ASV, and transfers the read medical care information from the communication I/F 4B to the vaccinated person information management server ASV.

### (2-4) Response Server CSV

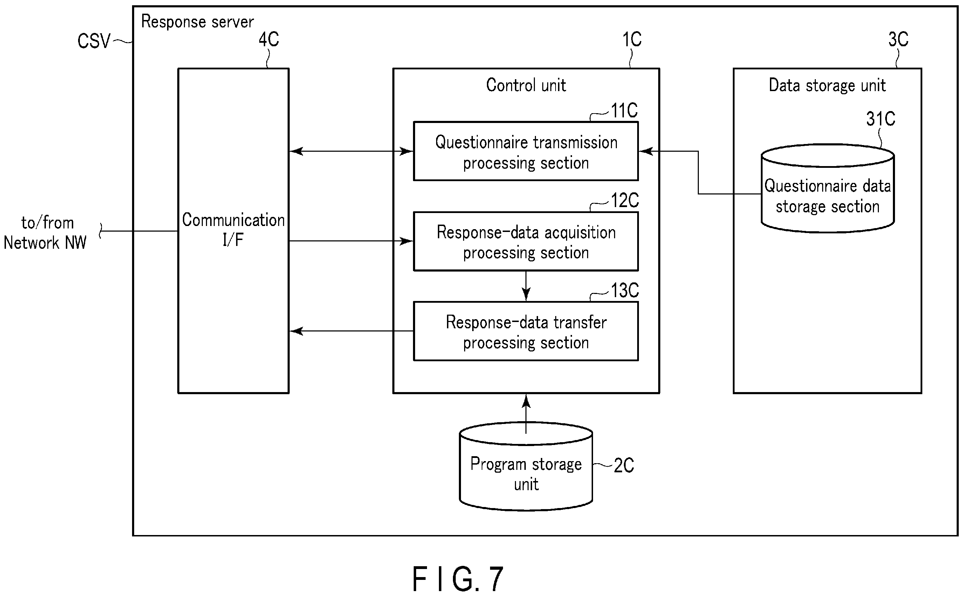

FIG. 7 is a block diagram showing an example of a software configuration of the response server CSV. Since the hardware configuration is the same as the configuration of the vaccinated person information management server ASV (FIG. 4), the description thereof is omitted here.

The response server CSV also includes a control unit 1C, a program storage unit 2C, a data storage unit 3C, and a communication I/F 4C. The communication I/F 4C performs transmission and reception of information data between the vaccinated person terminal UT and the vaccinated person information management server ASV via the network NW.

The data storage unit 3C includes a questionnaire data storage section 31C. The questionnaire data storage section 31C stores data as a questionnaire input sheet prepared in advance for each type of vaccine, for example.

The control unit 1C includes, as processing functions according to the embodiment of the present invention, a questionnaire transmission processing section 11C, a response-data acquisition processing section 12C, and a response-data transfer processing section 13C. Each of these processing sections 11C to 13C is realized by causing the hardware processor of the control unit 1C to execute a program stored in the program storage unit 2C.

A part or all of the processing sections 11C to 13C may be realized by using hardware such as an LSI or an ASIC.

In a case of receiving an instruction of a questionnaire from the vaccinated person information management server ASV, the questionnaire transmission processing section 11C transmits a request for response to the questionnaire together with a URL of the access destination to the corresponding vaccinated person terminal UT based on target person information SS5 received together with the instruction.

In a case where the URL is accessed by the vaccinated person terminal UT, the questionnaire transmission processing section 11C reads out the questionnaire data corresponding to the type of the vaccine from the questionnaire data storage section 31C. Then, the questionnaire transmission processing section 11C transmits the read questionnaire data together with consent inquiry information for confirming whether there is a consent from the communication I/F 4C to the corresponding vaccinated person terminal UT.

The response-data acquisition processing section 12C receives via the communication I/F 4C the response data SS6 input based on the template data of the questionnaire and the consent confirmation information SS3, and transfers the received response data SS6 and consent confirmation information SS3 to the response-data transfer processing section 13C.

The response-data transfer processing section 13C performs processing of returning, to the vaccinated person information management server ASV of the request source, the response data SS6 and the consent confirmation information SS3 transferred from the response-data acquisition processing section 12C.

### (Operation Example)

Next, an operation example of the system configured as described above will be described.

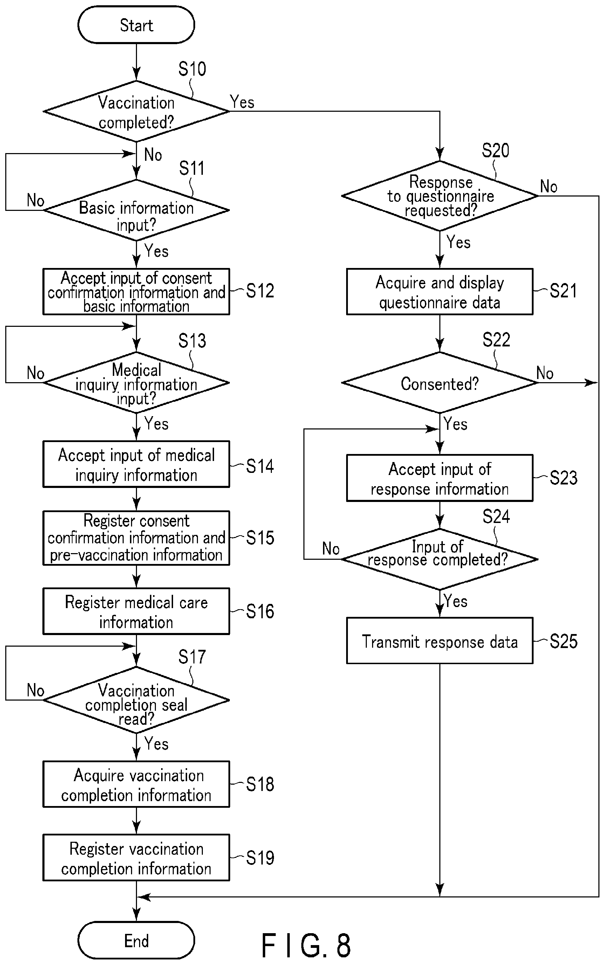

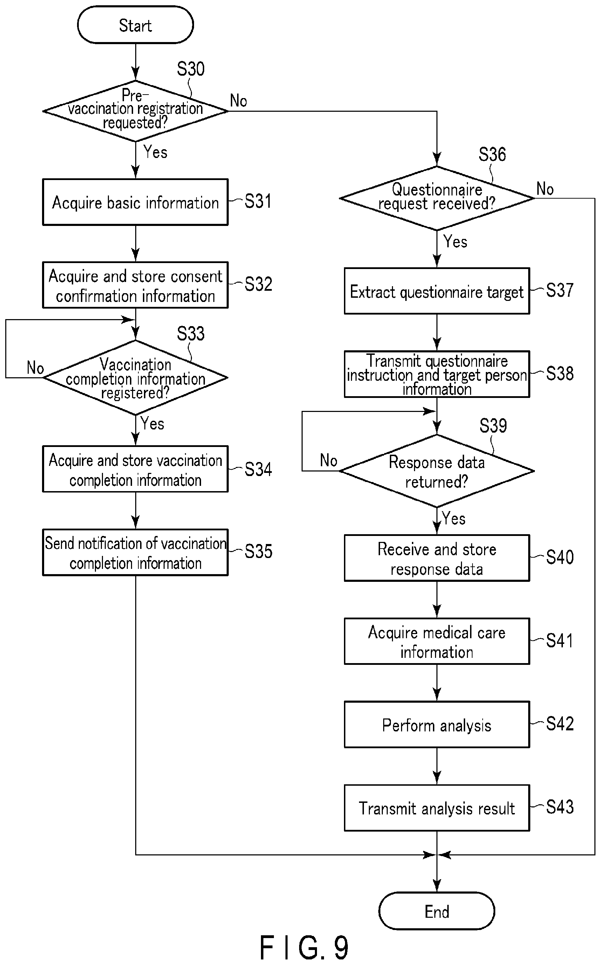

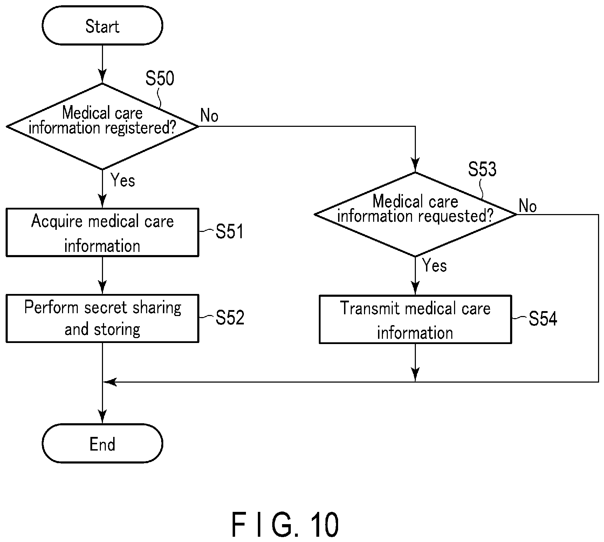

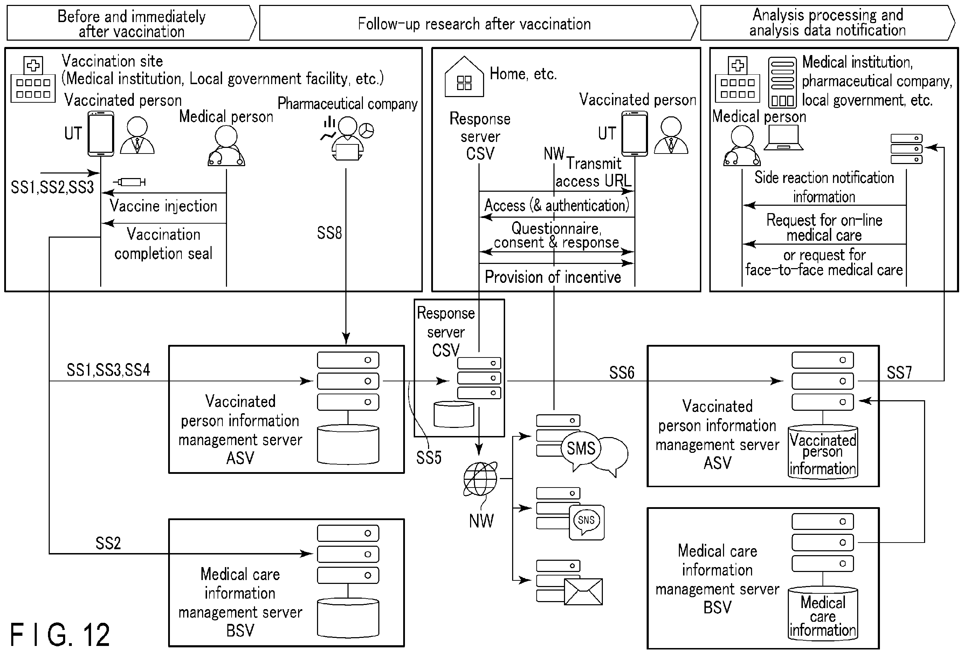

FIGS. 8 to 11 are flowcharts showing examples of procedures and details of processing by the vaccinated person terminal UT, the vaccinated person information management server ASV, the medical care information management server BSV, and the response server CSV, respectively. FIG. 12 is a sequence diagram showing an example of a data flow of the entire system.

### (1) Registration of Vaccinated Person Information and Medical Care Information

### (1-1) Registration of Basic Information SS1, Medical Inquiry Information SS2, and the like of Vaccinated Person

In a case where a person is vaccinated, side reactions considered to be harmful, such as anaphylaxis, may occur within a few hours after the vaccination, depending on the type of vaccine. Therefore, it is desirable that registration of the basic information SS1 is completed before or immediately after the vaccination.

The vaccinated person first activates an application for information registration in his/her own vaccinated person terminal UT and accesses the vaccinated person information management server ASV. At that time, authentication processing of the vaccinated person is first performed between the vaccinated person information management server ASV and the vaccinated person terminal UT.

Upon completion of the authentication processing, the control unit 1D of the vaccinated person terminal UT determines whether or not the vaccination has been completed in step S10. This determination is performed, for example, by confirming whether or not the reading of the vaccination completion information SS4 from the vaccination completion seal distributed after the completion of the vaccination has been completed.

When the vaccinated person selects and operates, for example, a basic information input button of the information registration application before vaccination, the control unit 1D of the vaccinated person terminal UT detects the operation of the basic information input button in step S11 under the control of the basic-information input acceptance processing section 11D, and then proceeds to step S12. In this step S12, the basic-information input acceptance processing section 11D acquires template data for inputting basic information from the vaccinated person information management server ASV and displays the template data on the display unit of the input/output device 6D. In a case where the template data is stored in advance in the vaccinated-person-information storage section 31D of the data storage unit 3D, the template data may be read out from the vaccinated-person-information storage section 31D and displayed on the display unit.

In this state, the vaccinated person inputs his/her own basic information SS1 at the input unit of the input/output device 6D. As the basic information SS1, for example, a unique vaccinated person identification number that the vaccinated person is notified of in advance, attribute information such as the name, age, and address of the vaccinated person, and contact information such as a phone number or mail address are input; however, the basic information is not limited thereto, and any addition or deletion can be made as necessary.

When the input operation is completed and a registration button is operated, the basic-information input acceptance processing section 11D fetches the input basic information SS1 via the input/output I/F 5D and temporarily stores the information in the vaccinated-person-information storage section 31D in step S12.

Under the control of the consent-information management processing section 16D, the control unit 1D of the vaccinated person terminal UT reads consent explanation information from the consent-information storage section 33D and causes the display unit of the input/output device 6D to display the read information. In this state, when the vaccinated person confirms the contents of the consent explanation information and inputs whether the vaccinated person has consented or not, the consent-information management processing section 16D stores the input information indicating whether the vaccinated person has consented or not in the consent-information storage section 33D as consent confirmation information SS3.

Upon completion of the input acceptance processing for the basic information SS1 and the consent confirmation information SS3, the control unit 1D of the vaccinated person terminal UT reads out the template data for inputting the medical inquiry information from the medical-care-information storage section 32D under the control of the medical-inquiry-information input acceptance processing section 12D, and causes the read data to be displayed on the display unit of the input/output device 6D. The template data may be acquired from the medical care information management server BSV and displayed.

In this state, the vaccinated person inputs the medical inquiry information SS2 in accordance with the template data. As the medical inquiry information SS2, for example, the current body temperature, whether the vaccinated person has a chronic disease, the type of a prescribed medicine, whether the vaccinated person has an allergy to a medicine, whether the vaccinated person is pregnant, a change in physical condition in a predetermined period in the past, and the like are input; however, the information to be input is not limited thereto.

If the input operation for the medical inquiry information SS2 is completed and the registration button is pressed, the medical-inquiry-information input acceptance processing section 12D proceeds from step S13 to step S14, fetches the input medical inquiry information SS2 via the input output I/F 5D, and stores the information in the medical-care-information storage section 32D in association with the corresponding vaccinated person identification number.

Upon completion of all of the input acceptance processing for the basic information SS1, the consent confirmation information SS3, and the medical inquiry information SS2, the control unit 1D of the vaccinated person terminal UT next reads out the basic information SS1 and the consent confirmation information SS3 respectively from the vaccinated-person-information storage section 31D and the consent-information storage section 33D in step S15, under the control of the information registration processing section 14D. Then, the information registration processing section 14D transmits the read basic information SS1 and consent confirmation information SS3 together with a request for pre-vaccination registration from the communication I/F 4D to the vaccinated person information management server ASV.

Upon detection of the request for pre-vaccination registration in step S30, under the control of the vaccinated-person-information acquisition processing section 11A, the control unit 1A of the vaccinated person information management server ASV receives the basic information SS1 via the communication I/F 4A in step S31 and causes the received basic information SS1 to be stored in association with the vaccinated person identification number in the vaccinated-person-information storage section 31A. In addition, in step S32, the vaccinated-person-information acquisition processing section 11A receives the consent confirmation information SS3 and causes the received consent confirmation information SS3 to be stored in the vaccinated-person-information storage section 31A in association with the vaccinated person identification number.

At this time, when storing the basic information SS1 and the consent confirmation information SS3, the vaccinated-person-information acquisition processing section 11A performs secret sharing on at least the basic information SS1 and causes the concealed basic information SS1 to be stored in the vaccinated-person-information storage section 31A. The secret sharing is, for example, processing of fragmenting the acquired target information into a plurality of pieces of meaningless data. Thus, even if the fragmented data is illegally acquired, the original target information cannot be restored. The secret sharing may also be performed on the consent confirmation information SS3.

Upon completion of the registration of the basic information SS1 and the consent confirmation information SS3, the information registration processing section 14D subsequently reads out the medical inquiry information SS2 from the medical-care-information storage section 32D in step S16. Then, the information registration processing section 14D transmits the read medical inquiry information SS2 to the medical care information management server BSV from the communication I/F 4D together with a request for medical care information registration in association with the vaccinated person identification number.

In a case where the PHR information, the prescription/medication information described in the electronic medication notebook, and the like of the vaccinated person are stored in advance in the medical-care-information storage section 32D, the information registration processing section 14D may transmit these pieces of information as the medical care information together with the medical inquiry information SS2. At this time, if vital data such as the current body temperature, blood pressure, heart rate, and the like measured by a biosensor built in or attached to the vaccinated person terminal UT is added to the PHR information, it is possible to accurately report the physical condition of the person to be vaccinated immediately before the vaccination.

On the other hand, if the request for medical care information registration is received in step S50, under the control of the medical-care-information acquisition processing section 11B, the control unit 1B of the medical care information management server BSV receives the medical care information including the medical inquiry information SS2, the PHR information, and the prescription/medication information via the communication I/F 4B in step S51. Then, in step S52, the medical-care-information acquisition processing section 11B causes the received medical care information to be stored in the medical-care-information storage section 31B of the data storage unit 3B in association with the vaccinated person identification number. At this time, the medical-care-information acquisition processing section 11B performs secret sharing on the medical care information and causes the concealed medical care information to be stored in the medical-care-information storage section 31B.

### (1-2) Registration of Vaccination Completion Information SS4

At the vaccination site, a vaccination completion seal is issued from a medical person or a person in charge in a local government to the person who has been vaccinated. On the vaccination completion seal, vaccination completion information SS4 is written using, for example, a bar code or a QR code. The vaccination completion information SS4 includes, for example, the type of the inoculated vaccine, the identification information (manufacturer ID) of the pharmaceutical company, and the lot number, but may also include the vaccinated person identification number, the vaccination date and time, the vaccination place, and the like.

The vaccinated person reads the vaccination completion information SS4 indicated on the vaccination completion seal using his/her own vaccinated person terminal UT. In this case, when the reading application is activated, the control unit 1D of the vaccinated person terminal UT proceeds from step S17 to step S18, and under the control of the vaccination-completion-information reading processing section 13D, the vaccination completion information SS4 written on the vaccination seal or sheet is optically read by the camera 7D. Then, the vaccination-completion-information reading processing section 13D fetches the read vaccination completion information SS4 via the input/output I/F 5D, and additionally stores the fetched information in the vaccinated-person-information storage section 31D in association with the vaccinated person identification number.

Upon completion of the processing of reading the vaccination completion information SS4, the control unit 1D of the vaccinated person terminal UT reads the vaccination completion information SS4 from the vaccinated-person-information storage section 31D in step S19 under the control of the information registration processing section 14D. Then, the information registration processing section 14D transmits the read vaccination completion information SS4 together with the request for registration from the communication I/F 4D to the vaccinated person information management server ASV.

On the other hand, the control unit 1A of the vaccinated person information management server ASV monitors the reception of the request for registration of the vaccination completion information SS4 in step S33. In this state, if the request for registration is received, under the control of the vaccinated-person-information acquisition processing section 11A, the control unit 1A receives the vaccination completion information SS4 via the communication I/F 4A in step S34, and causes the received vaccination completion information SS4 to be additionally stored in the vaccinated-person-information storage section 31A in association with the vaccinated person identification information. At this time, the vaccination completion information SS4 may also be concealed by the secret sharing.

### (1-3) Notification of Vaccination Completion Information SS4 to Pharmaceutical Company or Local Government

Upon completion of the registration of the vaccination completion information SS4, the control unit 1A of the vaccinated person information management server ASV performs processing of notifying the pharmaceutical company system PS or the governmental institution system GS of the vaccination completion information SS4 stored in the vaccinated-person-information storage section 31A in step S35 under the control of the vaccination-completion-information notification processing section 12A.

For example, the vaccination-completion-information notification processing section 12A first reads the consent confirmation information SS3 from the vaccinated-person-information storage section 31A, and determines whether or not the vaccinated person has consented to the notification of the vaccination completion information SS4 to the pharmaceutical company or the local government based on the read consent confirmation information SS3. As a result of this determination, if it is determined that the vaccinated person has consented, then the vaccination-completion-information notification processing section 12A reads the vaccination completion information SS4 of the vaccinated person from the vaccinated-person-information storage section 31A, and transmits the read vaccination completion information SS4 from the communication I/F 4A to the administrator terminal of the pharmaceutical company system PS or the governmental institution system GS registered in advance.

As a result, the pharmaceutical company or the local government can manage the type and lot number of the inoculated vaccine, the vaccination date and time, and the like for the vaccinated person.

### (2) Follow-up Research after Vaccination

### (2-1) Extraction of Targets of Research

In a case of conducting follow-up research after the vaccination, a questionnaire request SS8 is transmitted from, for example, the administrator terminal of the pharmaceutical company to the vaccinated person information management server ASV as shown in FIG. 12. The questionnaire request SS8 includes information indicating the extraction conditions, for example, information indicating the type of the inoculated vaccine and the vaccination date (vaccination period). In addition to the vaccination period, the extraction conditions may include information indicating a vaccination site, an age group, sex, and the like of the vaccinated person based on the basic information SS1, and may further include information such as whether the vaccinated person has an allergy or a previous disease based on the medical care information.

The control unit 1A of the vaccinated person information management server ASV monitors the reception of the questionnaire request SS8 in step S36. In this state, if the questionnaire request SS8 is received, the control unit 1A of the vaccinated person information management server ASV extracts from the vaccinated-person-information storage section 31A the vaccinated persons who meet the extraction conditions included in the questionnaire request SS8 in step S37 under the control of the questionnaire-target extraction processing section 13A. Then, in step S38, the questionnaire-target extraction processing section 13A transmits the target person information SS5 indicating the list of the extracted vaccinated persons to be the questionnaire targets together with the questionnaire instruction from the communication I/F 4A to the response server CSV. In the target person information SS5, for example, address information included in the basic information SS1 and information indicating the type of the inoculated vaccine are inserted for each subject.

The above target person extraction processing may be executed not only in response to the questionnaire request SS8 from the pharmaceutical company as a trigger, but also autonomously by the vaccinated person information management server ASV according to preset extraction conditions, for example.

### (2-2) Execution of Questionnaire Research

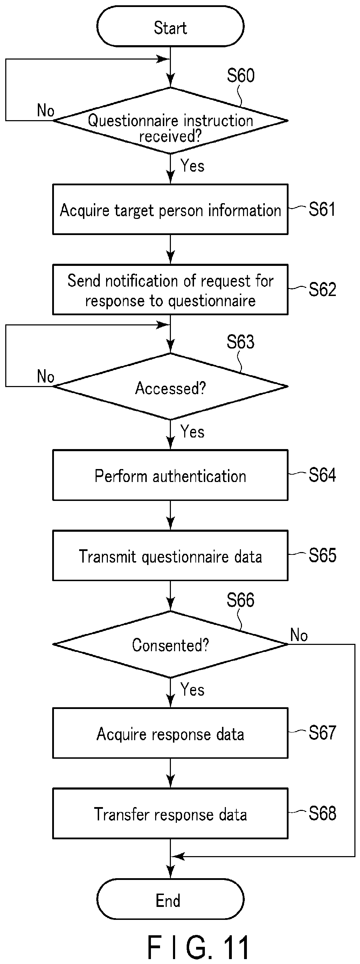

As shown in FIG. 11, if the questionnaire instruction is received in step S60, the control unit 1C of the response server CSV receives the target person information SS5 in step S61 under the control of the questionnaire transmission processing section 11C. Then, based on the received target person information SS5, the questionnaire transmission processing section 11C notifies the corresponding vaccinated person terminal UT of a request for response to the questionnaire in step S62. At this time, the request for response to the questionnaire includes a message requesting a response to the questionnaire and the URL of the response server CSV to be accessed. For example, email, SNS, or SMS is used as means for sending a notification of the request for response.

On the other hand, the control unit 1D of the vaccinated person terminal UT monitors the reception of the request for response to the questionnaire in step S20. If of the request for response to the questionnaire is received, the message and the access destination URL described in the received request for response are displayed on the display unit of the input/output device 6D.

In this state, it is assumed that the vaccinated person confirms the displayed message and clicks the access destination URL. Then, the browser of the vaccinated person terminal UT is activated to access the questionnaire response page represented by the URL of the response server CSV. On the other hand, if the access is detected in step S63, the control unit 1C of the response server CSV first authenticates the vaccinated person in step S64. Subsequently, in step S65, the questionnaire data corresponding to the type of the vaccine as a research target is read out from the questionnaire data storage section 31C, and the questionnaire data thus read out is transmitted from the communication I/F 4C to the vaccinated person terminal UT as the access source together with consent inquiry information.

Under the control of the questionnaire response processing section 15D, the control unit 1D of the vaccinated person terminal UT first receives the questionnaire data and the consent inquiry information in step S21, and causes the display unit of the input/output device 6D to display the consent inquiry information first. In this state, when the vaccinated person confirms the content of the consent inquiry information and inputs information indicating consent to the response to the questionnaire, the questionnaire response processing section 15D causes the consent-information storage section 33D to store the consent confirmation information SS3 input by the consent-information management processing section 16D and returns the information to the response server CSV.

Upon completion of the consent confirmation procedure described above, the control unit 1D of the vaccinated person terminal UT causes the display unit of the input/output device 6D to display the questionnaire data in step S23 under the control of the questionnaire response processing section 15D. Then, if the vaccinated person inputs the response data SS6 according to the displayed questionnaire data and pushes a transmission button, the questionnaire response processing section 15D proceeds from step S24 to step S25, and transmits the input response data SS6 from the communication I/F 4D to the response server CSV.

On the other hand, the control unit 1C of the response server CSV first confirms in step S66 the consent confirmation information SS3 sent from the vaccinated person terminal UT under the control of the response-data acquisition processing section 12C. If the consent of the vaccinated person is confirmed, the response-data acquisition processing section 12C receives in step S67 the response data sent from the vaccinated person terminal UT via the communication I/F 4C, and transfers the received response data SS6 to the response-data transfer processing section 13C. In step S68, the response-data transfer processing section 13C returns the response data SS6 to the vaccinated person information management server ASV together with the identification number of the vaccinated person who has responded.

If the response data SS6 is returned from the response server CSV, the control unit 1A of the vaccinated person information management server ASV proceeds from step S39 to step S40, receives the returned response data SS6 via the communication I/F 4A under the control of the response-data acquisition processing section 14A, and causes the response data storage section 32A to store the received response data SS6 in association with the identification number of the vaccinated person who has responded. At this time, the response data SS6 is concealed by the secret sharing and then stored.

### (2-3) Provision of Incentive

Upon confirmation of the consent of the vaccinated person in step S66, the control unit 1C of the response server CSV performs processing of providing an incentive to the vaccinated person who has consented to a use of the response data SS6. The incentive provision information is transmitted from the response server CSV to the vaccinated person terminal UT and managed by the response server CSV or the vaccinated person information management server ASDV. The incentive may be provided, for example, as points operated in various commercial services registered for use by the vaccinated person, but any other form such as cash payment may be adopted.

### (3) Analysis of Response Data SS6 and Notification of Analysis Data SS7

When the response data SS6 is acquired, the control unit 1A of the vaccinated person information management server ASV first transmits a request for acquisition of medical care information of the corresponding vaccinated person to the medical care information management server BSV in step S41 under the control of the analysis processing section 15A. On the other hand, if the request is received, the control unit 1B of the medical care information management server BSV proceeds from step S53 to step S54, and reads out the medical care information of the corresponding vaccinated person from the medical-care-information storage section 31B under the control of the medical-care-information transmission processing section 12B. Then, the medical-care-information transmission processing section 12B transmits the read-out medical care information to the vaccinated person information management server ASV of the request source.

Upon acquisition of the response data SS6 and the medical care information, the control unit 1A of the vaccinated person information management server ASV proceeds to step S42, and performs analysis processing on the response data SS6 by the analysis processing section 15A with reference to the medical care information. For example, the analysis processing section 15A first estimates whether or not a side reaction has occurred or is suspected to have occurred in the vaccinated person from the contents of the response of the plurality of items in the response data SS6. Subsequently, if it is estimated that a side reaction has occurred or is suspected to have occurred, the analysis processing section 15A determines whether or not the side reaction is harmful to the vaccinated person with reference to the information on whether the vaccinated person has a previous disease or allergy, the most recent health condition, and the like included in the medical care information. If the side reaction is determined to be harmful, the analysis processing section 15A estimates the necessity of on-line medical care or face-to-face medical care based on the content of the response data SS6.

In the analysis processing described above, the analysis processing section 15A performs the analysis processing on the concealed response data and the concealed medical care data on which the secret sharing process has been performed by using the secret calculation processing without restoring the concealed data to the original data.

When the estimation result is obtained, the control unit 1A of the vaccinated person information management server ASV proceeds to step S43. Then, under the control of the analysis-result transmission processing section 16A, the control unit 1A transmits analysis data SS7 including the estimation result from the communication I/F 4A to, for example, the administrator terminal of the medical institution system HS or the administrator terminal of the pharmaceutical company system PS. The analysis data SS7 may be transmitted to the administrator terminal of the governmental institution system GS.

Thus, the person in charge in the medical institution, the pharmaceutical company, or the local government is notified of a post-vaccination status of the vaccinated person. The person in charge can perform statistic processing related to the side reactions caused by the vaccinations based on the analysis data SS7, and can quickly respond to a request for on-line medical care or face-to-face medical care.

### (Functions and Effects)

As described above, in the embodiment, the basic information SS1, the consent confirmation information SS3, and the medical inquiry information SS2 of the vaccinated person are first registered in the vaccinated person information management server ASV and the medical care information management server BSV from the vaccinated person terminal UT before vaccination. Then, the vaccination completion information SS4 described on the vaccination completion seal distributed after the vaccination is read by the vaccinated person terminal UT and registered in the vaccinated person information management server ASV.

In the embodiment, when follow-up research for vaccinated persons is conducted, the vaccinated person information management server ASV extracts vaccinated persons corresponding to designated extraction conditions and notifies the response server CSV of the extracted vaccinated persons, the response server CSV transmits questionnaire data to the vaccinated person terminals UT, and the response server CSV acquires the response data SS6 to the questionnaire from the vaccinated person terminals UT.

Further, in the embodiment, the vaccinated person information management server ASV performs analysis processing for estimating whether a side reaction has occurred and whether on-line medical care or face-to-face medical care is necessary with reference to the medical care information of the vaccinated person registered in the medical care information management server BSV with respect to the response data SS6 acquired by the response server CSV, and notifies the medical institution, the pharmaceutical company, the local government, or the like of the analysis data SS7.

Therefore, according to the embodiment, for example, even for a vaccinated person whose medical record is not managed in a medical institution or the like, the basic information SS1, the consent information, and the medical inquiry information SS2 of the vaccinated person can be acquired and managed by the platform by using the vaccinated person terminal UT. Furthermore, the vaccination completion information SS4 can be acquired and managed by the platform by using the vaccinated person terminal UT. Therefore, it is possible to conduct follow-up research after vaccination for a wide range of the vaccinated persons.

In addition, by transmitting and receiving the questionnaire data and the response data SS6 to the questionnaire by using general-purpose communication means, such as email, SNS and SMS between the response server CSV and the vaccinated person terminals UT, it is possible to obtain a large amount of response data SS6 in a short period of time at a necessary follow-up timing without imposing a heavy burden on the vaccinated persons.

Then, the support control apparatus PF performs analysis processing on the acquired response data SS6 in real time, so that it is possible to efficiently analyze the post-vaccination situations of a greater number of vaccinated persons within an appropriate period, and to output the results of the analysis. For this reason, the medical institution, the pharmaceutical company, or the local government can perform statistical processing relating to side reactions of a vaccine, on-line medical care, face-to-face medical care, or the like at an appropriate timing.

### (Other Embodiments)

(1) In the embodiment described above, the basic information SS1, the consent confirmation information SS3, the medical inquiry information SS2, and the vaccination completion information SS4 are registered in the support control apparatus PF from the vaccinated person terminal UT carried by the vaccinated person. However, some vaccinated persons do not have their own portable terminal, or even if they have it, they may not be familiar with handling operations. In preparation for such a case, a terminal for information registration such as a tablet terminal may be prepared at the vaccination site, and a person in charge of support may perform a registration operation on behalf of the vaccinated person. In this case, if the terminal for information registration is provided with a function of optically reading the basic information SS1, the consent confirmation information SS3, and the medical inquiry information SS2 written on the paper medium by the vaccinated person, the basic information SS1, the consent confirmation information SS3, the medical inquiry information SS2, and the vaccination completion information SS4 of the vaccinated person can be registered in the support control apparatus PF.
(2) In the embodiment described above, when the response data SS6 indicating the post-vaccination situation is collected from the vaccinated person terminal UT, a request for response is transmitted from the support control apparatus PF to the vaccinated person terminal UT, a questionnaire is distributed in response to the access to the URL described in the response request by the vaccinated person terminal UT, and the response data SS6 is received. However, for example, instead of transmitting a request for response from the support control apparatus PF, the vaccinated person terminal UT may autonomously access a predetermined URL of the support control apparatus PF and transmit response data SS6 arbitrarily input by the vaccinated person to the support control apparatus PF from the vaccinated person terminal UT.
   In this way, for example, the vaccinated person can autonomously report the response data SS6 at a time point when his/her condition changes without waiting for reception of a request for response from the support control apparatus PF. In this case, the support control apparatus PF can soon recognize the occurrence of a sudden side reaction, such as anaphylaxis, of the vaccinated person after the vaccination, and can start on-line medical care by, for example, connecting the vaccinated person terminal UT and a doctor terminal of the medical institution.
(3) In the embodiment described above, the support control apparatus (platform) PF is configured by the vaccinated person information management server ASV, the medical care information management server BSV, and the response server CSV. However, the functions of these servers may be realized by a single server.
(4) In the embodiment described above, the case where the condition of the vaccinated person after the vaccination is followed up and researched has been described as an example. However, the present invention is not limited to this case and can be applied to a case where pharmaceutical companies or the like conduct clinical trials in the course of development of new medicines or the like other than vaccines. That is, the type of medicine is not limited to a vaccine, and other medicines may be used.
(5) In addition, the configuration and function of the support control apparatus (platform), the procedures and details of processing, the type and function of the vaccinated person terminal, and the like can be variously modified without departing from the scope of the present invention.

Although the embodiment of the present invention has been described in detail in the foregoing, the description is merely an example of the present invention in all of its aspects. Various improvements and modifications can be made without departing from the scope of the present invention. In other words, a specific configuration according to an embodiment may be adopted as appropriate when implementing the present invention.

In short, the present invention should not be limited to the above-described embodiments as-is, but may be embodied by modifying the components without departing from the scope of the invention at the implementation stage. In addition, various inventions can be made by suitably combining the structural elements disclosed in connection with the above embodiments. For example, some structural elements may be deleted from all the structural elements described in the embodiments. Furthermore, structural elements over different embodiments may be appropriately combined.

### REFERENCE SIGNS LIST

PF: support control apparatus (platform)
ASV: vaccinated person information management server
BSV: medical care information management server
CSV: response server
UT1 to UTn: vaccinated person terminals
HS: medical institution system
PS: pharmaceutical company system
GS: governmental institution system
NW: network
1A, 1B, 1C, 1D: control unit
2A, 2B, 2C, 2D: program storage unit
3A, 3B, 3C, 3D: data storage unit
4A, 4B, 4C, 4D: communication I/F
5D: input/output I/F
6D: input/output device
7D: camera
11A: vaccinated-person-information acquisition processing section
12A: vaccination-completion-information notification processing section
13A: questionnaire-target extraction processing section
14A: response-data acquisition processing section
15A: analysis processing section
16A: analysis-result transmission processing section
11B: medical-care-information acquisition processing section
12B: medical-care-information transmission processing section
11C: questionnaire transmission processing section
12C: response-data acquisition processing section
13C: response-data transfer processing section
11D: basic-information input acceptance processing section
12D: medical-inquiry-information input acceptance processing section
13D: vaccination-completion-information reading processing section
14D: information registration processing section
15D: questionnaire response processing section
16D: consent-information management processing section
31A, 31D: vaccinated-person-information storage section
32A: response data storage section
31B, 32D: medical-care-information storage section
31C: questionnaire data storage section
33D: consent-information storage section

## Claims

1. A patient follow-up research support system for supporting follow-up research on a patient who has received administration of a medicine, the patient follow-up research support system including a terminal device for information registration and a support control apparatus capable of communicating information data with the terminal device via a network,
the terminal device including:
an administration-completion-information registration processing section configured to accept an input of administration completion information issued to the patient who has received the administration of the medicine and to transmit the administration completion information to the support control apparatus together with unique identification information for identifying the patient; and
a response processing section configured to transmit, to the support control apparatus together with the unique identification information, response information that represents a condition of the patient after administration of the medicine and that is input in response to a request for response transmitted from the support control apparatus,
the support control apparatus including:
a reception processing section configured to receive the administration completion information transmitted from the terminal device and to store the received administration completion information in association with the unique identification information;
a response request processing section configured to extract, based on the administration completion information and a preset extraction condition, the patient corresponding to the extraction condition as a research target person and to transmit the request for response to the terminal device corresponding to the extracted research target person;
a response acquisition processing section configured to receive the response information transmitted from the terminal device in response to the request for response and to store the received response information; and
an analysis processing section configured to estimate a condition change of the patient after the administration of the medicine based on the response information and to output an estimation result.

2. The patient follow-up research support system according to claim 1, wherein
the terminal device further includes a medical-care-information registration processing section configured to accept an input of medical care information including medical inquiry information related to the patient to receive administration of the medicine, and to transmit the medical care information to the support control apparatus together with the unique identification information of the patient, and
the response request processing section is configured to add the medical care information to the administration completion information, to extract the patient corresponding to the extraction condition as the research target person, and to transmit questionnaire information corresponding to contents of the administration completion information and the medical care information together with the request for response to the terminal device corresponding to the extracted research target person.

3. The patient follow-up research support system according to claim 2, wherein
the terminal device further includes a consent confirmation information registration processing section configured to accept an input of consent confirmation information of the patient with respect to a use of at least one of the administration completion information, the medical care information, and the response information by the support control apparatus, and to transmit the consent confirmation information to the support control apparatus together with the unique identification information of the patient, and
the response request processing section is configured to extract the patient corresponding to the extraction condition as the research target person from among patients whose consent has been confirmed based on the consent confirmation information.

4. A support control apparatus for supporting follow-up research on a patient who has received administration of a medicine and capable of communicating information data with a terminal device for information registration via a network, the support control apparatus comprising:
an administration-completion-information acquisition processing section configured to acquire administration completion information issued to the patient who has received the administration of the medicine from the terminal device together with unique identification information for identifying the patient;
a response request processing section configured to extract, based on the administration completion information and a preset extraction condition, the patient corresponding to the extraction condition as a research target person and to transmit a request for response to the terminal device corresponding to the extracted research target person;
a response acquisition processing section configured to receive response information transmitted from the terminal device in response to the request for response and to store the received response information; and
an analysis processing section configured to estimate a condition change of the patient after the administration of the medicine based on the response information and to output an estimation result.

5. The support control apparatus according to claim 4, further comprising a medical-care-information acquisition processing section configured to acquire medical care information including medical inquiry information related to the patient to receive administration of the medicine from the terminal device together with the unique identification information of the patient, wherein
the response request processing section is configured to add the medical care information to the administration completion information, to extract the patient corresponding to the extraction condition as the research target person, and to transmit questionnaire information corresponding to contents of the administration completion information and the medical care information together with the request for response to the terminal device corresponding to the extracted research target person.

6. The support control apparatus according to claim 4, wherein the analysis processing section is configured to estimate whether or not a harmful reaction has occurred in the patient based on the response information.

7. The support control apparatus according to claim 4, wherein the analysis processing section is configured to estimate whether on-line medical care or face-to-face medical care for the patient is necessary based on the response information.

8. The support control apparatus according to claim 4, further comprising an incentive provision processing section configured to execute consent confirmation processing with respect to use of the response information by the support control apparatus with the terminal device, and to execute processing of providing an incentive to the patient who has received the administration of the medicine and has consented to the use.

9. A terminal device for information registration capable of communicating information data via a network with a support control apparatus configured to perform processing for supporting follow-up research on a patient who has received administration of a medicine, the terminal device comprising:
an administration-completion-information registration processing section configured to accept an input of administration completion information issued to the patient who has received the administration of the medicine and to transmit the administration completion information to the support control apparatus together with unique identification information for identifying the patient; and
a response processing section configured to transmit, to the support control apparatus together with the unique identification information, response information that represents a condition of the patient after the administration of the medicine and that is input in response to a request for response transmitted from the support control apparatus.

10. The terminal device according to claim 9, wherein the administration-completion-information registration processing section is configured to acquire the administration completion information by reading a two-dimensional code written on an administration completion sheet issued to the patient who has received the administration of the medicine.

11. A patient follow-up research support method performed by a patient follow-up research support system for supporting follow-up research on a patient who has received administration of a medicine, the patient follow-up research support system including a terminal device for information registration and a support control apparatus capable of communicating information data with the terminal device via a network, the method comprising:
accepting, by the terminal device, an input of administration completion information issued to the patient who has received the administration of the medicine, and transmitting the administration completion information to the support control apparatus together with unique identification information for identifying the patient;
receiving, by the support control apparatus, the administration completion information transmitted from the terminal device and storing the received administration completion information in association with the unique identification information;
extracting, by the support control apparatus, based on the administration completion information and a preset extraction condition, the patient corresponding to the extraction condition as a research target person and transmitting a request for response to the terminal device corresponding to the extracted research target person;
transmitting, by the terminal device to the support control apparatus together with the unique identification information, response information that represents a condition of the patient after the administration of the medicine and that is input in response to the request for response transmitted from the support control apparatus;
receiving, by the support control apparatus, the response information transmitted from the terminal device and storing the received response information; and
estimating, by the support control apparatus, a condition change of the patient after the administration of the medicine based on the response information, and outputting an estimation result.

12. A program storage medium storing a program that causes a processor included in the support control apparatus according to claim 4 to execute processing by each of the processing sections included in the support control apparatus.

13. A program storage medium storing a program that causes a processor included in the terminal device according to claim 9 to execute processing by each of the processing sections included in the terminal device.
